# EUROPEAN PATENT APPLICATION

(11) **EP 1 891 949 A1**
(43) Date of publication of application: **27.02.2008**
(21) Application number: 06016934.9
(22) Date of filing: 14.08.2006
(51) Int. Cl.: A61K 31/404, A61P 21/00

(54) **Use of tricyclic indole derivatives for the treatment of muscular atrophy**

(71) Applicant: Santhera Pharmaceuticals (Schweiz) AG, 4410 Liestal (CH)
(72) Inventor: Deppe, Holger, 4053 Basel (CH); Briguet, Alexandre, 4125 Riehen (CH); Metz, Günther, 69221 Dossenheim (DE)
(74) Representative: Grünecker, Kinkeldey, Stockmair & Schwanhäusser Anwaltssozietät

(57) **Abstract**

The present invention relates to the use of a compound for the preparation of a medicament for the treatment of muscular atrophy.

## Description

The present invention relates to the use of a compound for the preparation of a medicament for the prophylaxis and/or treatment of muscular atrophy.

A general loss of skeletal muscle mass is a characteristic, debilitating response to fasting, as well as many severe diseases, including advanced cancer, renal failure, sepsis, and diabetes. In addition, atrophy of specific muscles results from their disuse or denervation. In most types of muscle atrophy overall rates of protein synthesis are suppressed and rates of protein degradation are consistently elevated; this response accounts for the majority of the rapid loss of muscle protein. In a variety of animal models of human diseases e.g., fasting, diabetes, cancer cachexia , acidosis, sepsis , disuse atrophy , denervation , and glucocorticoid treatment, most of the accelerated proteolysis in muscle appears to be due to an activation of the Ub-proteasome pathway. This activation is associated with the upregulation of muscle specific ligases.

Muscle specific ubiquitin ligases are an essential part of the ubiquitin-proteasome pathway for protein degradation. Indeed, increased expression of the ubiquitin ligases MAFbx and MURF1 is a critical component in muscle atrophy. These so called atrogins are upregulated in a rat atrophy model (Gomes et al., PNAS 98, 2001, 14440-14445). Moreover, mice deficient of MAFbx, which is sometimes also referred to as atrogin1, or MURF1 are resistent to atrophy (Bodine et al., Science 294, 2001, 1704-1708). FOXO transcription factors induce are essential to upregulate the atrophy-related ubiquitin ligases MAFbx and MURF and thus contribute to developing skeletal muscle atrophy (Goldberg et al., Cell 117, 2004, 399-412). Skeletal muscle FOX01 transgenic mice have less skeletal muscle mass, down-regulated Type I (slow twitch/red muscle) fiber genes, and impaired glycemic control. (Kamei et al., J. Biol. Chem. 279, 2004, 41114-411123).

FOXO transcription factors belong to the large Forkhead family of proteins, a family of transcriptional regulators characterized by a conserved DNA-binding domain termed the 'forkhead box'. The Forkhead family is present in all eukaryotes. In humans, the FOXO Forkhead subgroup contains four members (FOXO1, FOXO3, FOXO4, and FOX06). The transcriptional activity of FOXO transcription factors is regulated via its acetylation level. Whereas the majority of data describe that acetylation of FOXO factors represses target gene transcription, some suggest that FOXO acetylation increases target gene transcription. This discrepancy has been attributed to cell type and the target-gene-specific effects of FOXO. (Greer et al., Oncogene 24, 2005, 7410-7425).

Protein acetylation regulates a wide variety of cellular functions, including the recognition of DNA by proteins, protein-protein interactions, and protein stability. Post-translational modification of proteins at lysine residues by reversible acetylation is catalyzed by the opposing activities of histone acetyltransferases (HATs) and histone deacetylases (HDACs), which act on both histone and non-histone substrates despite their names. HDACs are grouped into three classes on the basis of their homology to yeast transcriptional repressors. The class III HDACs are homologous to the yeast transcriptional repressor Sir2p and have no sequence similarity to class I and II HDACs; these Sir2 proteins are also called sirtuins (Denu, Curr. Opin. Chem. Biol. 9, 2005, 431-440).

Sirtuins have been found in bacteria to eukaryotes. The hallmark of the family is a domain of approximately 260 amino acids that has a high degree of sequence similarity in all sirtuins. The family is divided into five classes (I-IV and U) on the basis of a phylogenetic analysis of 60 sirtuins from a wide array of organisms. Class I and class IV are further divided into three and two subgroups, respectively. The U-class sirtuins are found only in Gram-positive bacteria. The human genome encodes seven sirtuins, with representatives from classes I-IV.

Non-histone substrates of the most studied human Sir2 homolog, SIRT1, were shown to include p53, FOXO proteins, p300, NFkB and PGC-1a, implicating sirtuins in apoptosis, cell survival, transcription and metabolism. Data indicate that SIRT1 is one of multiple deacetylases involved in the regulation of FOXO dependent transcription in mammalian cells (Brunet et al., Science 303, 2004, 2011-2015).

Atrophy is a common form of muscle tissue loss which can be long-lasting but, nevertheless, is frequently reversible. The classic example would be the muscle deterioration seen in bed ridden patients but genetically determined diseases such as for spinal muscular atrophy can cause life-threatening muscle atrophy as well.

Loss of muscle tissue is also observed as a consequence of cancer, cardiac disease, infectious disease, chronic obstructive pulmonary disease (COPD), rheumatic disease, inflammatory bowel disease, end-stage renal disease, liver cirrhosis, severe injury, where it is referred to as cachexia.

Finally, severe and life threatening loss of muscle tissue is observed in genetic diseases causing muscle dystrophies exemplified in Duchenne Muscular Dystrophy, limb girdle muscular dystrophies and congenital muscle dystrophies.

Preventing loss of muscle tissue or accelerating the recovery from muscle weakness and/or loss due to atrophy, cachexia or dystrophy would be of great clinical benefit and highly desirable since the current treatment options are very limited. Therapeutic options for muscle atrophy include physiotherapy and similar interventions such as electro-stimulation of affected muscle groups, while pharmacological interventions available to prevent muscle loss or accelerate recovery from muscle weakness are not at all satisfying. Those agents include appetite stimulants such as megestrol, fish oil and certain unsatured fatty acids and canabinoid-like substances.

It is the object of the present invention to provide a compound for the treatment of muscular atrophy which has superior properties compared to known compounds conventionally used in the therapy of muscular atrophy, such as a better efficacy and improved patient compliance.

Said object is achieved by the use of tricyclic indole derivatives according to formula (I) for the preparation of a medicament for the prophylaxis and/or treatment of muscular atrophy.

### Brief description of the drawings

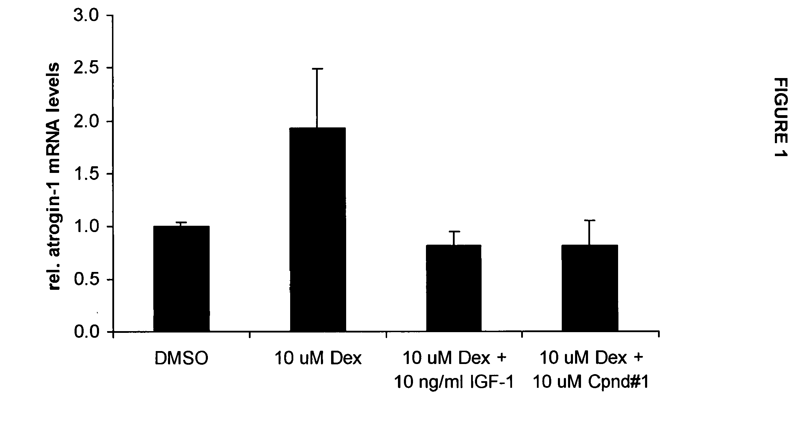

Figure 1 shows a histogram representing the mRNA levels measured in cultured C2C12 myotubes treated for 24 h with solvent only (1 % DMSO), 10 µM Dex, 10 µM Dex and 10 ng/ml IGF-1 or 10 µM Dex and 10 µM Compound 1.

The invention relates to the use of a compound according to formula (I): 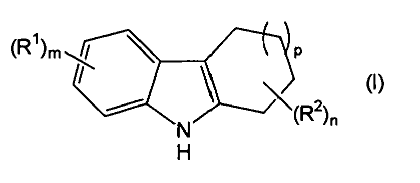 wherein
each of R¹ and R² is, independently from each other, halo, hydroxy, C₁-C₁₀ alkyl, C₁-C₆ haloalkyl, C₁-C₁₀ alkoxy, C₁-C₆ haloalkoxy, Z-C₆-C₁₀ aryl, Z-C₅-C₁₀ heteroaryl, Z-C₃-C₈ heterocyclyl, Z-C₃-C₈ cycloalkyl, C₂-C₁₂ alkenyl, C₂-C₁₂ alkynyl, Z-C₅-C₁₀ cycloalkenyl, Z-C₅-C₁₀ heterocycloalkenyl, carboxy, carboxylate, cyano, nitro, amino, C₁-C₆ alkyl amino, C₁-C₆ dialkyl amino, mercapto, S(O)NH₂, S(O)₂NH₂, C₁-C₄ alkylenedioxy, oxo, acyl, aminocarbonyl, C₁-C₁₀ alkoxycarbonyl, hydrazinocarbonyl, C₁-C₆alkyl hydrazinocarbonyl, C₁-C₆ dialkyl hydrazinocarbonyl, hydroxyamino carbonyl, or alkoxyamino carbonyl, wherein each alkyl, heterocyclyl, cycloalkyl and cycloalkenyl is optionally substituted by one or more R³; and wherein each aryl and heteroaryl is optionally substituted by one or more R⁴;
R³ is independently from each other fluoro, methyl, methoxy, hydroxy or amino;
R⁴ is independently from each other fluoro, chloro, methyl, methoxy, cyano, hydroxy, amino, CF₃, CHF₂, CH₂F, OCF₃, OCHF₂ or OCH₂F;
Z is a bond or C₁-C₆ alkylene, wherein the C₁-C₆ alkylene group is optionally substituted with one or more fluorine atoms;
m is 1, 2, 3 or 4;
n is 1, 2, 3 or 4; and
p is 0, 1, 2 or 3
or its tautomeric, enantiomeric, diastereomeric or pharmaceutically acceptable salts thereof
for the preparation of a medicament for the prophylaxis and/or treatment of muscular atrophy.

Compounds of formula (I) preferably used in the present invention are those compounds in which one or more of the residues contained therein have the meanings given below. It is understood, that the preferably used compounds cover any compound obtained by combining any of the definitions disclosed within this description for the various substituents. With respect to all compounds of the formulas (I) the present invention also includes all tautomeric and stereoisomeric forms and mixtures thereof in all ratios, and their pharmaceutically acceptable salts.

In preferred embodiments of the present invention, the substituents R¹ and R² as well as the indices m, n and p of the formula (I) independently from each other have the following meaning. Hence, one or more of the substituents R¹ and R² as well as the indices m, n and p can have the preferred or more preferred meanings given below.

In a preferred embodiment of the present invention, R¹ is halo, C₁-C₆ alkyl, Z-C₆-C₁₀ aryl, Z-C₅-C₁₀ heteroaryl or Z-C₃-C₈ cycloalkyl, wherein alkyl, aryl, heteroaryl and cycloalkyl are optionally substituted with one or more substituents R³. More preferably, R¹ is halo, preferably chloro, or Z-cyclopropyl which is unsubstituted or substituted with one or more substituents R³.

R² is as defined above. Preferably, R² is aminocarbonyl.

It is further preferred that each alkyl, alkenyl, alkynyl, cycloalkyl, heterocyclyl, heterocycloalkenyl or cycloalkenyl group in the definition of R¹ and R² is unsubstituted or substituted by 1 to 4 substituents R³, more preferably 1 to 3 substituents R³, most preferably 1 to 2 substituents R³. In one preferred embodiment, two substituents R³ are simultaneously attached to the same carbon atom of the alkyl, aryl, heteroaryl, cycloalkyl, heterocyclyl or cycloalkenyl group.

It is further preferred that each aryl or heteroaryl group in the definition of R¹ and R² is unsubstituted or substituted by 1 to 4 substituents R⁴, more preferably 1 to 3 substituents R⁴, most preferably 1 to 2 substituents R⁴.

R³ is as defined as above. Preferably, R³ is fluoro, methyl or methoxy. Most preferably, R³ is fluoro. In the case of a Z-C₃-C₈ cycloalkyl group, R³ is preferably attached to the carbon atom connected to the tricyclic ring system if Z is a bond or to the carbon atom connected to the alkylene chain if Z is a C₁-C₆ alkylene group.

R⁴ is as defined above. Preferably, R⁴ is fluoro, methyl, methoxy, hydroxy, amino or CF₃.

Z is as defined above. Preferably, Z is a bond or a C₁-C₂ alkylene optionally substituted with 1 to 4 fluoro.

In a preferred embodiment, Z is -CF₂-, -CH₂-CF₂-, -CH₂-CH₂-CF₂- or -CH₂-CH₂-CH₂-CF₂-, wherein the substituted alkyl chain is connected with the tricyclic moiety via the CF₂ group.

The index m is as defined above. Preferably, m is 1, 2 or 3, more preferably, m is 1 or 2. If m = 1 then the substituent R¹ is preferably located on position 6 of the tricyclic indole ring system.

The index n is as defined above. Preferably, n is 1, 2 or 3, more preferably, n is 1 or 2. In a preferred embodiment, n is 1 and R² is attached to position 1 of the ring.

The index p is as defined above. Preferably, p is 1 or 2.

Where tautomerism, like e.g. keto-enol tautomerism, of compounds of general formula (I), may occur, the individual forms, like e.g. the keto and enol form, and together as mixtures in any ratio are also within the scope of the invention. Same applies for stereoisomers, like e.g. enantiomers, cis/trans isomers, conformers and the like.
Preferred embodiments of the compounds according to present invention are shown below.

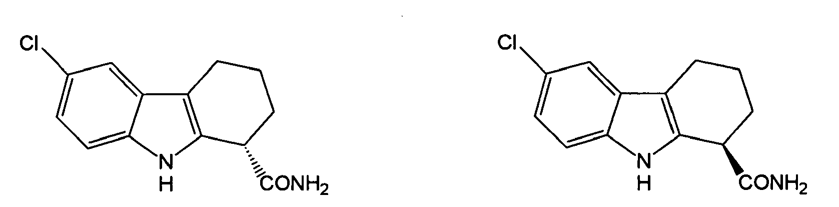

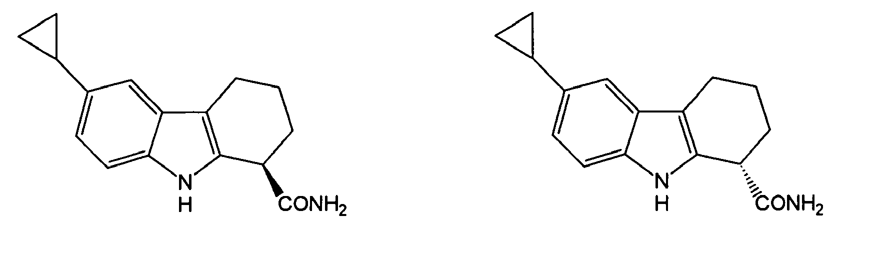

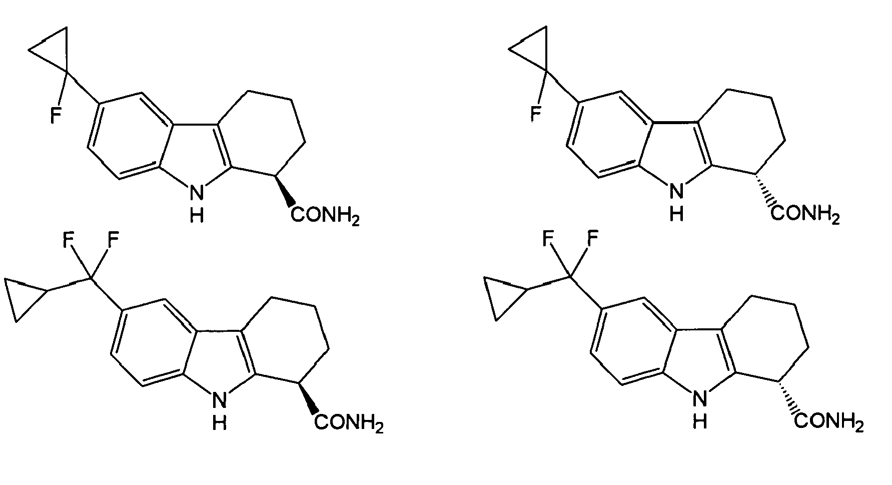

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions).

Within the meaning of the present invention the terms are used as follows:
"C₁-C₁₀ Alkyl" means a straight-chain or branched carbon chain having 1 - 10 carbon atoms, e.g. methyl, ethyl, n-propyl, isopropyl, n-butyl, isobutyl, sec-butyl, tert-butyl, n-pentane, n-hexane, n-heptane, n-octane, n-nonane or n-decane. Each hydrogen of a C₁-C₁₀ alkyl carbon may be replaced by a substituent.
"C₂-C₁₂ Alkenyl" means a straight-chain or branched carbon chain of 2- 12 carbon atoms having one or more double bonds in the chain. Examples of C₂-C₁₂ alkenyl include -CH=CH₂, -CH=CH-CH₃, -CH₂-CH=CH₂, -CH=CH-CH₂-CH₃, -CH=CH-CH=CH₂, -CH₂-CH₂-CH₂-CH=CH₂, -CH₂-CH₂-CH₂-CH₂-CH=CH₂, -CH₂-(CH2)₄-CH=CH₂, -CH₂-(CH₂)₅-CH=CH₂, -CH₂-(CH₂)₆-CH=CH₂, -CH₂-(CH₂)₇-CH=CH₂, -CH₂-(CH₂)₈-CH=CH₂ and -CH₂-(CH₂)₉-CH=CH₂. Each hydrogen of a C₂-C₁₂ alkenyl carbon may be replaced by a substituent.
"C₂-C₁₂ Alkynyl" means a straight-chain or branched carbon chain of 2- 12 carbon atoms having one or more triple bonds in the chain. Examples of C₂-C₁₂ alkynyl include -C1≡CH, -C≡C-CH₃, -CH₂-C≡CH, -C≡CH₂-CH₃, -C≡C-C≡CH, -CH₂-CH₂-CH₂-C≡CH, -CH₂-CH₂-CH₂-CH₂-C≡CH, -CH₂-(CH₂)₄-C≡CH, -CH₂-(CH₂)₅-C≡CH, -CH₂(CH₂)₆-C≡CH, -CH₂-(CH₂)₇-C≡CH, -CH₂-(CH₂)₈-C≡CH and -CH₂-(CH₂)₉-C≡CH. Each hydrogen of a C₂-C₁₂ alkynyl carbon may be replaced by a substituent.
"C₃-C₈ Cycloalkyl" as employed herein includes saturated monocyclic hydrocarbon group having 3 to 8 carbons., e.g. cyclopropyl, cyclobutyl, cyclopentyl, cyclohexyl, cycloheptyl or cyclooctyl. Each hydrogen of a C₃-C₈ cycloalkyl carbon may be replaced by a substituent.
"C₅-C₁₀ Cycloalkenyl" means a cyclic alkyl chain having 5 - 11 carbon atoms, which is partially unsaturated e.g. cyclopentenyl, cyclopentadienyl, cyclohexenyl, cycloheptenyl, cyclooctenyl, cyclononenyl or cyclodecenyl. Each hydrogen of a C₅-C₁₀ cycloalkenyl carbon may be replaced by a substituent.
"Halo" means fluoro, chloro, bromo or iodo. It is generally preferred that halogen is fluoro or chloro.
"C₃-C₈ Heterocyclyl" refers to a nonaromatic 3-8 membered monocyclic ring system having 1-3 heteroatoms selected from O, N, or S. Any ring atom can be substituted. Examples of C₃-C₈ heterocyclyl include tetrahydrofuranyl, tetrahydropyranyl, piperidinyl, morpholino, imidazolidinyl and pyrrolidinyl.
"C₅-C₁₀ Heterocycloalkenyl" means a partially saturated, nonaromatic 5-10 membered monocyclic ring system having 1-3 heteroatoms selected from O, N, or S. Any ring atom can be substituted. Examples of C₅-C₁₀ heterocycloalkenyl include tetrahydropyridyl and dihydropyranyl.
"C₆-C₁₀ Aryl" refers to an aromatic monocyclic or bicyclic hydrocarbon ring system, wherein any ring atom capable of substitution can be substituted. Examples of C₆-C₁₀ aryl moieties include phenyl and naphthyl.
"C₅-C₁₀ Heteroaryl" refers to an aromatic 5-10 membered monocyclic or bicyclic ring system having 1-3 heteroatoms selected from O, N, N(O) or S. Any ring atom can be substituted. Examples of C₅-C₁₀ Heteroaryl include
"Acyl" means an alkylcarbonyl, cycloalkylcarbonyl, arylcarbonyl, heterocyclylcarbonyl, or heteroarylcarbonyl substituent, any of which may be further substituted.

The terms "aminocarbonyl," "C₁-C₁₀ alkoxycarbonyl", "hydrazinocarbonyl", and "hydroxyaminocarbonyl" refer to the radicals -C(O)NH, -C(O)O-C₁-C₁₀ alkyl, -C(O)NHNH₂ and -C(O)NH₂NH₂, respectively.

The terms " C₁-C₆ alkylamino" and " C₁-C₆ dialkylamino" refer to -NH-C₁-C₆ alkyl and -N(C₁-C₆ alkyl)₂ radicals, respectively.

The term "alkoxy" refers to an -O- C₁-C₁₀ alkyl radical. The term "mercapto" refers to an SH radical.

Pharmacological agents presently available to prevent muscle loss or accelerate recovery from muscle weakness are not at all satisfying. Those agents include appetite stimulants such as megestrol, fish oil and certain unsatured fatty acids and canabinoid-like substances. An alternative approach to reduce atrophy, cachexia or dystrophy or to improve recovery from atrophy is offered by interventions that slow or stop the degradation of muscle proteins which frequently is mediated by the ubiquitin-proteasome system. Activation of this proteolytic enzyme complex as seen in clinical conditions of atrophy, cachexia and dystrophy critically depends on the ligation of peptides and proteins to ubiquitin moieties. Atrogins are a class of muscle-specific ligases that couple target proteins and peptides with ubiquitin moieties thereby mediating proteolysis of muscle proteins.

A method for treatment of muscular atrophies is offered by identifying pharmacological agents that would appropriately modulate any of the molecules involved in the signaling cascade that leads to pathologically increased muscle protein degradation. Specifically, a solution to this problem would be offered by identifying pharmacological modulators of either of the known atrogins or, alternatively, pharmacological modulators of a suitable member of upstream activators of atrogin expression. The identification of such pharmacological modulators offers a broad commercial application since atrophy, cachexia and dystrophy is common to a number of chronic disease states characterized by long periods of immobility. There is also a potential to apply this treatment approach in a subset of the geriatric population.

Surprisingly, it has been found, that inhibitors of sirtuins downregulate atrogin levels in a model for muscle atrophy in cells. Without being bound to a theory it is contemplated that the treatment of differentiated myotubes with an inhibitor of SIRT1 results in the suppression of atrogin-1 mRNA levels. As discussed above, muscular atrophy is associated with the upregulation of muscle specific ligases, e.g. atrogins, therefore, inhibitors of sirtuins can be used for the treatment of muscular atrophies.

The compounds of formula (I) have been previously described as inhibitors of sirtuins and as such have been proposed for the treatment of various diseases such as cancer, diabetes, Parkinson's or Alzheimer's disease or obesity (e.g. US-A-2005/0209300). It has now been shown for the first time that tricyclic indol derivatives exhibit an unexpectedly high activity in the modulation of atrogin levels. Said compounds therefore are potential drugs for treating muscular atrophy.

In practical use, the compounds of formula (I) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) are administered orally.

The effective dosage of active ingredient employed may vary depending on the particular compound employed, the mode of administration, the condition being treated and the severity of the condition being treated. Such dosage may be ascertained readily by a person skilled in the art.

Preferably, the effective dosage is chosen in a range between 0.01 milligram to about 100 milligram per kilogram of mammal body weight, preferably given as a single daily dose or in divided doses two to six times a day, or in sustained release form. More preferably, the dosage is between 0.01 milligram to about 50 milligram per kilogram of animal body weight. This dosage regimen may be adjusted to provide the optimal therapeutic response.

The following example illustrates the invention but is not intended to limit the invention in any way.

### Example

### Atrogin-1 levels in C2C12 myotubes

For the determination of *atrogin-1* mRNA levels, C2C12 myoblasts were seeded at a density of 40'000 cells per well in 6-well plates previously coated with 0.1 % gelatin. The cells were then grown for 2 days in growth medium containing 20% fetal calf serum. To initiate myotube differentiation, the growth medium was replaced by fusion medium containing 5% horse serum and the cells were incubated for another 4 days until myotubes formed.

Differentiated myotubes were then pre-treated with 10 ng/ml IGF-1, 10 µM Compound 1 (6-Chloro-2,3,4,9-tetrahydro-1H-carbazo)e-1-carboxylic acid amide) or solvent only (1% DMSO) for 1 hour. After this pre-incubation, myotube atrophy was induced by adding 10 µM of the glucocorticoid dexamethasone (Dex) and the cultures were incubated for another 24 hours.

At the end of this treatment the cultures were rinsed once with PBS and total RNA was extracted. For each culture, 0.5 µg RNA were then reversed transcribed and *atrogin-1* mRNA levels were determined relative to the 18 S mRNA levels using real-time PCR. For each treatment condition *atrogin-1* mRNA levels were quantified in three separate cultures. The results are shown in Figure 1.

As was also shown by others (Sandri et al., Cell 117, 2004, 399-412) treatment of differentiated myotubes with the glucocorticoid dexamethasone (Dex) induces *atrogin-1* mRNA levels 2-fold. This increase in *atrogin-1-m*RNA levels can be suppressed by co-treatment with 10 ng/ml of IGF-1 to the level measured in solvent treated myotubes. IGF-1 is a protein which acts through the IGF-Akt pathway.

As can be taken from Figure 1, co-treatment of differentiated myotubes with compound 1 (6-Chloro-2,3,4,9-tetrahydro-1 H-carbazole-1-carboxylic acid amide, 10µM) for 1 h also suppresses the increase in *atrogin-1* mRNA in Dex treated myotubes to the level measured in solvent treated myotubes or IGF-1 treated myotubes.

## Claims

1. Use of a compound according to formula (1): 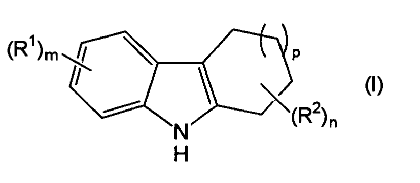 wherein
each of R¹ and R² is, independently from each other, halo, hydroxy, C₁-C₁₀ alkyl, C₁-C₆ haloalkyl, C₁-C₁₀ alkoxy, C₁-C₆ haloalkoxy, Z-C₆-C₁₀ aryl, Z-C₅-C₁₀ heteroaryl, Z-C₃-C₈ heterocyclyl, Z-C₃-C₈ cycloalkyl, C₂-C₁₂ alkenyl, C₂-C₁₂ alkynyl, Z-C₅-C₁₀ cycloalkenyl, Z-C₅-C₁₀ heterocycloalkenyl, carboxy, carboxylate, cyano, nitro, amino, C₁-C₆ alkyl amino, C₁-C₆ dialkyl amino, mercapto, S(O)NH₂, S(O)₂NH₂, C₁-C₄ alkylenedioxy, oxo, acyl, aminocarbonyl, C₁-C₁₀ alkoxycarbonyl, hydrazine-carbonyl, C₁-C₆ alkyl hydrazinocarbonyl, C₁-C₆ dialkyl hydrazinocarbonyl, hydroxyamino carbonyl, or alkoxyamino carbonyl, wherein each alkyl, alkenyl, alkynyl, heterocyclyl, heterocycloalkenyl, cycloalkyl and cycloalkenyl is optionally substituted by one or more R³; and wherein each aryl and heteroaryl is optionally substituted with one or more R⁴;
R³ is independently from each other fluoro, methyl, methoxy, hydroxy or amino;
R⁴ is independently from each other fluoro, chloro, methyl, methoxy, cyano, hydroxy, amino, CF₃, CHF₂, CH₂F, OCF₃, OCHF₂ or OCH₂F;
Z is a bond or C₁-C₆ alkylene, wherein the C₁-C₆ alkylene group is optionally substituted with one or more fluorine atoms;
mis1,2,3or4;
n is 1, 2, 3 or 4; and
p is 0, 1, 2 or 3
or its tautomeric, enantiomeric, diastereomeric or pharmaceutically acceptable salts thereof
for the preparation of a medicament for the prophylaxis and/or treatment of muscular atrophy.

2. The use according to claim 1, wherein R¹ is halo, C₁-C₆ alkyl, Z-C₆-C₁₀ aryl, Z-C₅-C₁₀ heteroaryl or Z-C₃-C₈ cycloalkyl, wherein alkyl, and cycloalkyl are optionally substituted with one or more R³ and wherein aryl and heteroaryl are optionally substituted with one or more R⁴.

3. The use according to claim 1 or 2, wherein R¹ is halo or Z-cyclopropyl optionally substituted with one or more R³.

4. The use according to any one of claims 1 to 3, wherein R² is SO₂NH₂ or aminocarbonyl.

5. The use according to any one of claims 1 to 4, wherein p is 1 or 2.

6. Use of sirtuin modulators for regulating atrogin levels in a mammal.

7. Use of sirtuin modulators for the preparation of a medicament for the prophylaxis and/or treatment of disorders associated with elevated atrogin levels.

8. The use according to claim 7 for the prophylaxis and/or treatment of muscular atrophy.
